Europäisches Patentamt

European Patent Office

Office européen des brevets

(11) Publication number: **0 313 375 B1**

## EUROPEAN PATENT SPECIFICATION

(45) Date of publication of patent specification: **04.08.93**   (51) Int. Cl.[5]: **C07C 1/04**, B01J 23/84

(21) Application number: **88309904.6**

(22) Date of filing: **21.10.88**

(54) **Process and catalyst for converting synthesis gas to hydrocarbons.**

(30) Priority: **23.10.87 US 113095**

(43) Date of publication of application:
**26.04.89 Bulletin  89/17**

(45) Publication of the grant of the patent:
**04.08.93 Bulletin  93/31**

(84) Designated Contracting States:
**AT BE CH DE ES FR GB GR IT LI LU NL SE**

(56) References cited:
**EP-A- 0 220 343**
**US-A- 3 449 078**

(73) Proprietor: **DEN NORSKE STATS OLJESEL-SKAP A.S.**
**Postboks 300 Forus**
**N-4001 Stavanger(NO)**

(72) Inventor: **Goodwin, James G., Jr.**
**24 Timberline Court**
**Pittsburgh, PA 15217(US)**
Inventor: **Marcelin, George**
**5853 Hobart Street**
**Pittsburgh, PA 15217(US)**
Inventor: **Eri, Sigrid**
**Venusveien 30E**
**N-7000 Trondheim(NO)**
Inventor: **Riis, Trygve**
**Holtegt. 8**
**N-0259 Oslo 2(NO)**

(74) Representative: **Rees, David Christopher et al**
**Kilburn & Strode 30 John Street**
**London WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

## Description

The present invention relates to a process and a catalyst for converting synthesis gas to hydrocarbons.

The reaction to convert carbon monoxide and hydrogen mixtures (defined herein as synthesis gas or syngas) to higher hydrocarbons over metallic catalysts has been known since the turn of the century. This reaction is commonly referred to as the Fischer-Tropsch or F-T synthesis. During World War II, Germany exploited a process employing the F-T synthesis for the production of gasoline and other hydrocarbon products. By 1944 a total of nine F-T plants were operating in Germany. The German process used primarily a catalyst composed of cobalt, magnesium oxide, thorium oxide and kieselguhr, in the relative proportions of 100:5:8:200. Later, most of the thoria was replaced by magnesia, primarily for economic reasons. Currently, commercial Fischer-Tropsch plants are operating in South Africa. These plants use a process employing a precipitated iron-based catalyst which contains various promoters to improve the stability and product distribution.

Common F-T catalysts are nickel, cobalt and iron. Nickel was probably the first substance to be recognised as capable of catalysing the reaction of syngas to hydrocarbons, producing mainly methane (see, for example, "The Fischer-Tropsch Synthesis" by R.B. Anderson, Academic Press (1984), p.2). Iron and cobalt are able to produce longer chain length hydrocarbons and are thus preferred as catalysts for the production of liquid hydrocarbons. However, other metals are also capable of catalysing the F-T synthesis. Ruthenium is a very active catalyst for the formation of hydrocarbons from syngas. Its activity at low temperatures is higher than that of iron, cobalt or nickel, and it produces a high proportion of heavy hydrocarbons. At high pressures, it produces a high proportion of high molecular weight waxes. Osmium has been found to be moderately active, while platinum palladium and iridium exhibit low activities (see Pichler, "Advances in Catalysts", vol. IV, Academic Press, N.Y. 1952). Other metals which are active, such as rhodium, yield high percentages of oxygenated materials (Ichikawa, Chemtech, 6, 74(1982)). Other metals that have been investigated include rhenium, molybdenum and chromium, but these exhibit very low activities with most of the product being methane.

Various combinations of metals can also be used for synthesis. Doping cobalt catalysts with nickel causes an increase in methane production during F-T synthesis (See "Catalysis", col IV, Reinhold Publishing Co., (1956), p.29). In U.S. Patent 4,088,671 to T.P. Kobylinski, entitled "Conversion of Synthesis Gas Using a Cobalt-Ruthenium Catalyst", the addition of small amounts of ruthenium to cobalt is shown to result in an active F-T synthesis catalyst with a low selectivity to methane. Thus, these references teach that the combination of two or more metals can result in an active F-T catalyst. In general, the catalysts of these teachings have activities and selectivities which are within the ranges of the individual components.

Combinations of metals with certain oxide supports have also been reported to result in an improved hydrocarbon yield during F-T synthesis, probably due to an increase in the surface area of the active metal. The use of titania to support cobalt or cobalt-thoria is taught in U.S. Patent 4,595,703 entitled "Hydrocarbons from Synthesis Gas". In this case the support serves to increase the activity of the metal(s) towards hydrocarbon formation. In fact, titanium belongs to a class of metal oxides known to exhibit strong metal-support interactions and, as such, has been reported to give improved F-T activity for a number of metals (see, for example S.J. Tauster et al, Science, 211, 1121 (1981)). Combinations of titania and two or more metals have also been shown to yield improved F-T activity. In U.S. Patent 4,568,663, entitled "Cobalt Catalysts in the Conversion of Methanol to Hydrocarbons and for Fischer-Tropsch Synthesis", combinations of cobalt, rhenium and thoria and cobalt and rhenium supported on titania are claimed useful for the production of hydrocarbons from methanol or synthesis gas. This, patent also indicates that similar improvements in activity can be obtained when cobalt-rhenium or cobalt-rhemium-thoria is compounded with other inorganic oxides. However, titania is the only support specifically discussed. The typical improvement in activity gained by promotion of cobalt metal supported on titania with rhenium is less than a factor of 2. It has been found by the present inventors that the addition of rhenium to cobalt metal supported on a number of other common supports results in similar improvements in activity.

The only other examples in the literature of catalysts involving mixtures of cobalt and rhenium refer to completely different chemical reactions. For example, in Soviet Union Patent 610558, a catalyst composed of cobalt and rhenium supported on alumina is taught to result in improved performance for the steam reforming of hydrocarbons. Steam reforming of ;hydrocarbons is a completely different process from hydrocarbon production via F-T synthesis and is believed to proceed by a completely different mechanism. Although some steam reforming catalysts can convert synthesis gas to hydrocarbons, such catalysts arte not selective for the productin of high carbon-number hydrocarbons ($C_3$ and above) during conversion of synthesis gas. In fact, most commonly used steam reforming catalysts contain nickel as their active metal, and nickel produces mostly methane when used for syngas conversion.

It has been found in accordance with the present invention that synthesis gas comprising hydrogen and carbon monoxide can be converted to liquid hydrocarbons by using a catalyst and a process employing a catalyst consisting of cobalt and rhenium supported on alumina and promoted with an alkali. As used herein, "alkali" refers to one or more of the elements lithium, sodium, potassium, rubidium and casium of Group IA of the periodic table. The catalyst preferably contains from 5 to 60% cobalt and has a rhenium content between 0.5 and 50% of the amount of cobalt and an alkali content between 0.5 and 5 atom% of the amount of cobalt. The alumnia preferably is gamma alumina.

It has been found that the addition of small amounts of rhenium to catalysts consisting predominantly of cobalt supported on alumina unexpectedly results in greatly enhanced activity of this catalyst of hydrocarbon production from syngas. This is surprising in light of the fact that rhenium supported on alumina shows very low activity, with most of the product being methane. Furthermore, rhenium addition to cobalt supported on supports other than alumina results in catalysts with much lower activity levels. In addition, the more active cobalt plus rhenium catalyst maintains the high selectivity to higher hydrocarbons and the low selectivity to methane found with alumina-supported cobalt catalyst. It has also been found that the addition of small amounts of an alkali to these catalysts serves to increase the average carbon number of the products produced during F-T synthesis.

Both the high activity and the low methane production of cobalt-rhenium on alumina are unexpected in the light of various facts. Firstly, rhenium shows very low activity for F-T synthesis. Secondly, the main products from F-T synthesis over rhenium catalyst are methane and carbon dioxide. Thirdly, the use of alumina as a support for catalysts containing only cobalt results in no, or at best only a slight, increase in activity compared to the use of cobalt on other supports. Thus, for reasons not fully understood, the combination of cobalt and rhenium plus an alkali supported on alumina results in a catalyst which is significantly more active than either of the two individual metals supported on alumina or the combination of the two metals supported on other inorganic supports, such as silica, magnesia, silica-alumina, titania, chromia or zirconia. Furthermore, the product distribution with a high selectivity to $C_2$ + hydrocarbons and low selectivity to methane and carbon dioxide could not have been predicted based on the known product distribution from rhenium catalysts. In addition, the addition of an alkali to the catalyst serves to increase the average carbon number of the product. This is advantageous in situations where the market value of the lighter products is lower than that of the heavier products.

The process includes the step of contacting a synthesis gas feed comprised of hydrogen and carbon monoxide over such a catalyst, or a catalyst comprising cobalt and rhenium composited on an alumina support where in rhenium is present in relatively lesser amounts than the cobalt content of the catalyst.

Preferred reaction conditions include a temperature in the range of 150 to 300°C, a pressure in the range of 1.01 to 101 bars (atmospheric to 100 atmospheres) and a gaseous hourly space velocity, based on the total amount of synthesis gas feed, in the range of 100 to 20,000 cm$^3$ of gas per gram of catalyst per hour.

The catalyst of the present invention therefore comprises, as the active catalytic ingredients, cobalt and rhenium supported on alumina with rhenium present in a relatively smaller amount than cobalt. Optionally, the catalyst can also comprise an alkali metal, that is an element from Group IA of the periodic table.

Operating conditions suitable for use in the process of this invention are a reaction temperature between 150 and 300°C, preferably between 180 and 280°C, and more preferably between 190 and 250°C; a total pressure from atmospheric to 100 atmospheres, preferably between 1.01 to 40.53 bars (1 and 40 atmospheres), and more preferably between 1.01 to 30.40 bars (1 and 30 atmospheres); and a gaseous hourly space velocity, based on the total amount of synthesis gas feed, between 100 and 10,000cm$^3$ of gas per gram of catalyst per hour, and preferably from 100 to 10,000 cm$^3$/g/h, where gaseous hourly space velocity is defined as the volume of gas (measured at standard temperature and pressure) fed per unit weight of catalyst per hour.

The catalyst used in the process of this invention has been found to be highly active for the conversion of synthesis gas, a mixture of hydrogen and carbon monoxide, into a mixture of predominantly paraffinic hydrocarbons. As indicated above, it has long been known that cobalt is an active catalyst for the F-T synthesis. It is also known that the addition of rhenium to a cobalt catalyst supported on titania gives improved activity, even if rhenium by itself shows very low activity for F-T synthesis and produces methane as the main product. Surprisingly, it has been found that the choice of support for the cobalt plus rhenium catalyst is very critical, and that the addition of rhenium to an alumia-supported cobalt catalyst gives a much higher improvement in activity than the addition of rhenium to cobalt supported on the other inorganic oxides. Also, the alkali increases the average carbon number of the product.

The cobalt added to the alumina support may be in an amount up to about 60wt% of the catalyst, including cobalt. Preferably amounts between 5 and 45 wt% are used, and more preferably, between 10 and 45wt%. The content of rhenium may be between 0.5 and 50wt% of the cobalt content; preferably between 1 and 20 wt%; and more preferably from 2 to 20 wt%. The content of the alkali is preferably between 0.5 and 5 atom % of the cobalt content.

In addition to cobalt, rhenium and alkali, it may be beneficial to include a small amount of a metal oxide promoter in an amount between 0.2 and 5wt% and more preferably between 0.2 and 2 wt%, based on the weight of the complete catalyst. The promoter is suitably chosen from elements in groups IIIB, IVB or VB of the periodic table, the lanthanides and the actinides. The promoter oxide can be chosen from, for example, $SC_2O_3$, $Y_2O_3$, $La_2O_3$, $Ce_2O_3$, $Pr_2O_3$, $ZrO_3$, $Ac_2O_3$, $PaO_2$, $Nd_2O_3$, $CeO_2$, $V_2O_5$, or $Nb_2O_5$. The most preferable oxide is $La_2O_3$, or a mixture of lanthanides, rich in lanthanum. Oxides like MnO or MgO can also by included. While not essential, the use of these metal oxides is common in the art, since they are believed to promote the production of products with higher boiling points, while maintaining or improving catalytic activity. However, the catalyst is highly active and selective without the addition of one or more of these metal oxide promoters.

Preferred embodiments of the invention will now be described by way of example.

THE CATALYST SUPPORT

The catalytically active metals, the alkali, and the promoter metal oxide, if present, are distended on alumina. It has been found that other supports, for example, silica, titania, chromia, magnesia, silica-alumina and zirconia, produce catalysts with much lower activities.

To be most effective when used as a support, alumina should be characterised by low acidity, high surface area, and high purity. These properties are preferred in order to enable the catalyst to have high activity and a low deactivation rate, and to produce high molecular weight hydrocarbon products. The surface area of the alumina support may be at least, and preferably greater than, about $100m^2/g$, and more preferably greater than $150m^2/g$. The pore volume may be at least, and preferably greater than, about $0.3cm^3/g$. The catalyst support is preferably of high purity. That is, the content of elements, e.g. sulphur and phosphorous, that have a deleterious effect on catalytic activity must preferably be kept low. The sulphur content of the catalyst support should be kept below 100ppm and more preferably below 50ppm. Although gamma alumina has generally been used and is preferred, a number of alumina structures, if prepared properly, can meet these conditions and are suitable supports. For example, eta-alumina, xi-alumina, theta-alumina, delta-alumina, kappa-alumina, boehmite and pseudo-boehmite can all be used as supports.

CATALYST PREPARATION

The method of depositing the active metals, the alkali, and the promoter oxide on the alumina support is not critical, and can be chosen from various methods well known to those skilled in the art. One suitable method that has been employed is known as incipient wetness impregnation. In this method the metal salts are dissolved in an amount of a suitable solvent just sufficient to fill the pores of the catalyst. In another method, the metal oxides or hydroxides are coprecipitated from an aqueous solution by adding a precipitating agent. In still another method, the metal salts are mixed with the wet support in a suitable blender to obtain a substantially homogeneous mixture. In the present invention, if incipient wetness impregnation is used, the catalytically active metals and the alkali can be deposited on the support using an aqueous or an organic solution. Suitable organic solvents include, for example, acetone, methanol, ethanol, dimethyl fomamide, diethyl ether, cyclohexane, xylene and tetrahydrofuran. Aqueous impregnation is preferred when $Co(NO_3)_2$ is used as the salt, while an organic solvent is the preferred solvent when the catalyst is prepared from cobalt carbonyl.

Suitable cobalt compounds include, for example, cobalt nitrate, cobalt acetate, cobalt chloride and cobalt carbonyl, with the nitrate being the most preferable when impregnating from an aqueous solution. Suitable rhenium compounds include, for example, rhenium oxide, rhenium chloride and perrhenic acid. Perrhenic acid is the preferred compound when preparing a catalyst using an aqueous solution. Suitable alkali salts for incorporating the alkali into the catalyst include, for example, the nitrates, chlorides, carbonates, and hydroxides. The metal oxide promoter can suitably be incorporated into the catalyst in the form of for example the nitrate or chloride.

After aqueous impregnation, the catalyst is dried at 110 to 120°C for 3 to 6 hours. When impregnating from organic solvents, the catalyst is preferably first dried in a rotary evaporator apparatus at 50 to 60°C under low pressure, then dried at 110 to 120°C for several hours longer.

The dried catalyst is calcined under flowing air by slowly increasing the temperature to an upper limit of between 200 and 500°C, preferably between 250 and 350°C. the rate of temperature increase is preferably between 0.5 and 2C° per minute, and the catalyst is held at the highest temperature for a period of 2 to 5 hours. The impregnation procedure is repeated as many times as necessary to obtain a catalyst with the desired metals content. Cobalt, rhenium and the promoter, if present, can be impregnated together, or in separate steps. If separate steps are used, the order of impregnating the active components can be varied.

Before use, the calcined catalyst is preferably reduced with hydrogen. This can suitably be done in flowing hydrogen at atmospheric pressure at a flow rate between 30 and $100cm^3$/min when reducing about 2g of catalyst. The flow rate should suitably be increased for larger quantities of catalyst. The temperature is increased at a rate between 0.5 and 2C° per minute from ambient to a maximum level of 250 to 450°C, preferably between 300 and 400°C, and maintained at the maximum temperature for 6 to 24 hours, more preferably 10 to 24 hours.

After the reduction step, the catalyst may be oxidised and reduced before use. To carry out the oxidation step, the catalyst is treated with dilute oxygen (1-3% oxygen in nitrogen) at room temperature for a period of 1/2 to 2 hours before the temperature is increased at the same rate and to the same temperature as used during calcination. After holding the high temperature for 1 to 2 hours, air is slowly introduced, and the treatment is continued under air at the high temperature for another 2 to 4 hours. The second reduction is carried out under the same conditions as the first reduction.

HYDROCARBON SYNTHESIS

The reactor used for the synthesis of hydrocarbons from synthesis gas can be chosen from various types well known to those skilled in the art, for example, fixed bed, fluidised bed, ebullating bed or slurry. Because of the exothermic nature of the F-T reaction, the reactor must be designed with heat removal capabilities so that the desired reaction temperature can be carefully controlled. The above listed reactor types have characteristics that made them well suited for use in the process of this invention. The catalyst particle size for the fixed or ebullating bed is preferably between 0.1 and 10mm and more preferably between 0.5 and 5mm. For the other types of reactors a particle size between 0.01 and 0.3 mm is preferred.

The synthesis gas used as feed to the process is a mixture of carbon monoxide and hydrogen and can be obtained from any source known to those skilled in the art, such as, for example, steam reforming of natural gas or partial oxidation of coal. The molar ratio of $H_2$:CO is normally between 0.5:1 to 3:1, preferably between 1:1 to 3:1, and more preferably between 1.5:1 to 2.5:1. Carbon dioxide is not a desired feed component for use with the process of this invention, but it does not adversely affect the process or the activity of the catalyst, other than acting as a diluent. All sulphur compounds must, on the other hand, be held to very low levels in the feed, preferably below 1ppm, because they have an adverse affect on the activity of the catalyst employed in this process.

A preferred process according to the present invention may be described as follows. Synthesis gas from any suitable source, as discussed previously, is fed to the process. The ratio of hydrogen to carbon monoxide in this synthesis gas may be between 1 and 3 and more preferably between 1.5 and 2.5. A highly desirable ratio is the stoichiometric ratio of $H_2$ to CO usage within the process, which is about 1.2 to 2.2. Providing syngas at this stoichiometric ratio results in the most efficient utilisation of the syngas in the process, since neither hydrogen nor carbon monoxide is in excess. In addition to $H_2$ and CO, the syngas may contain quantities of other gases, such as carbon dioxide, methane, and nitrogen. These gases act as diluents which may be disadvantageous for other reactor systems, as will be more fully discussed below. The hydrogen sulphide content of the syngas must be kept very low, preferably below 1ppm by volume, because sulphur is a severe catalyst poison.

If not at sufficient pressure, the syngas is compressed to process pressure, which can be from 1.01 to 101 bars (atmospheric to 100 atmospheres), preferably from 1.01 to 40.53 bars (1 to 40 atmospheres), and more preferably from 1.01 to 30.40 bars (1 to 30 atmospheres). Anything lower than 1 atmosphere would require operation at vacuum conditions which is not necessary and unduly expensive. The rate of reaction would decrease. Pressure greater than about 101 bars (100 atmospheres) would increase the cost significantly due to the increased strength of the equipment necessary to withstand high pressures. The syngas is then preheated before entering the reactor. Because the F-T reaction is highly exothermic, it is not normally necessary to heat the feed gas all the way to reaction temperature, the final heatup taking place in the reactor itself. As discussed previously, the reactor can suitably be chosen from a variety of reactor types, the most important criterion being the ability to control carefully the temperature of the exothermic F-T reaction. The three,. most suitable reactor types are tubular fixed bed reactors, in which the catalyst is placed in tubes and a fluid is circulated on the outside of the tubes for heat removal; fluidised bed reactors; and slurry reactors, in which finely divided catalyst is slurried in a vehicle oil. In these latter two reactor types, heat can be removed in a variety of ways, including increases in the sensible heat of the feed, internal heat exchangers, and removal of a slip stream for cooling and return to the reactor.

The temperature in the reactor should be between 150 and 325°C, and more preferably between 180 and 280°C. The total pressure can be from 1.01 to 101 bars (atmospheric to 100 atmospheres), preferably between 1.01 and 30.40 bars (1 and 30 atmospheres). These temperature ranges are typical for Fischer-Tropsch reactions. The rate of reaction at temperatures below 150°C would be so low as to be uneconomical on a commercial scale due to the large reactor size which would be required. Where temperatures above about 325°C are employed, the selectivity to liquid hydrocarbons is so low that the process becomes economically unfeasible. There are less expensive ways to make methane. Gaseous hourly space velocity (based only on the $H_2$ plus CO content of the feed) should be between 0.1 and 20 $m^3$ per kg catalyst per hour and preferably between 0.1 and $10m^3$ per kg catalyst per hour. Once the desired working temperature and pressure are determined, the space velocity is chosen to give the desired per pass conversion.

The acceptable form of the catalyst will depend upon the type of reactor being used. For tubular fixed bed reactors, the catalyst can be in the form of extrudates, pellets, spheres, granules, etc. with a nominal diameter of about 0.5 to 6mm, and preferably about 1.5mm. For fluidised bed or slurry reactors, the catalyst should be in finely divided form. A typical analysis for a catalyst suitable for slurry reactor operation is :

| Particle Diameter, Microns | Weight % of Sample |
|---|---|
| 0- 5 | 0.2 |
| 5- 7 | 1.5 |
| 7- 9 | 1.3 |
| 9- 13 | 0.5 |
| 13- 19 | 1.1 |
| 19- 27 | 1.5 |
| 27- 38 | 3.3 |
| 38- 53 | 5.7 |
| 53- 75 | 15.7 |
| 75-106 | 25.7 |
| 106-150 | 26.4 |
| 150-212 | 13.9 |
| 212-300 | 3.2 |

For either fluidised bed or slurry operation, it is important that the catalyst charge contain neither too large a fraction of large particles nor too large a fraction of small particles. A proper size content is important for successful fluidisation or suspension of the catalyst. In slurry operation, it is also important to have a proper vehicle. Normally, a fraction of the product oil will be used for this purpose. Generally, a carbon number distribution in the range from $C_{20}$ to $C_{50}$ is satisfactory. For startup purposes, in addition to a F-T liquid, $C_{30}$ to $C_{50}$ poly-alpha-olefins may be used or a highly refined, i.e. heteroatom and aromatic free, petroleum oil.

In the reactor, contact between the syngas and the catalyst results in the production of largely paraffinic hydrocarbons, along with small amounts of olefins and oxygenates. In general, part of the product will remain in the gas phase and be carried out of the reactor along with inerts and unconverted feed gas, and part of the product will form a liquid phase. The fraction of the product that is carried out in the vapour phase will depend upon the operating conditions being used.

In a fixed bed reactor, the liquid phase will either drain out the bottom of the catalyst tubes or else be entrained out with the offgas. In a fluidised bed reactor, operating conditions must be adjusted so that the amount of liquid produced is very low. Otherwise, the product builds up on the catalyst and destroys its fluidisation properties. In the slurry reactor, the liquid products will dissolve in the vehicle oil and can be recovered by any of the techniques familiar to those skilled in the art, such as filtration, centrifugation, settling and decanting, etc.

The offgas from the reactor is cooled to condense liquid products (both hydrocarbons and water, which is produced as a by product). This is typically done in a series of steps at progressively lower temperatures. This is necessary to prevent any waxes from solidifying and causing plugging. After the final cooler/separator, additionally hydrocarbons can be recovered by absorption or adsorption, if desired.

Per pass conversions in the reactor can vary from 10 to over 90%, preferably from 40 to 90%. If the once through conversion is not sufficiently high, the offgas, after product recovery and bleeding off of a slip steam to prevent inserts from building up in the system, can be mixed with the fresh syngas feed and recycled to the reactor.

As mentioned, the syngas may contain some quantity of nitrogen. For fixed bed operation, this may be undesirable since dilution with nitrogen reduces the partial pressure of reactive gases and increases the pressure drop. However, for fluidised bed and slurry reactor operation, the nitrogen may be beneficial by providing additional mixing energy which may help to keep the catalyst suspended. In these types of reactors, pressure drop is not a strong function of flow rate.

As a final step in the process, all the liquid products may be combined. If desired they may be stabilised by distillation to remove highly volatile components. The liquid product may then be marketed as a synthetic crude or alternatively, distilled into individual product cuts which can be marketed separately. As a further alternative the product may be catalytically dewaxed or hydrocracked before being marketed. These latter processes can improve product properties by lowering pour point, increasing octane number, and changing boiling ranges.

The products from this process are a complicated mixture, consisting predominantly of normal paraffins, but also containing small amounts of branched chain isomers, olefins, alcohols and other oxygenated compounds. The main reaction can be illustrated by the following equation:

$$nCo + 2nH_2 \longrightarrow (-CH_2-)_n + nH_2O$$

where $(-CH-)_n$ represents a straight chain hydrocarbon of carbon number n. The carbon number refers to the number of carbon atoms making up the main skeleton of the molecule. Products range in carbon number from one to 50 or higher.

In addition, with many catalysts, for example those based on iron, the water gas shift reaction is a well known side reaction:

$$CO + H_2O \longrightarrow H_2 + CO_2$$

With the catalyst used in the process of this invention, the rate of this last reaction is usually very low. However, it is found that, even though rhenium catalysts exhibit a relatively high selectivity to carbon dioxide, the cobalt plus rhenium catalyst used in the process of this invention surprisingly does not have a higher selectivity to carbon dioxide than the cobalt only catalyst.

The hydrocarbon products from processes employing the Fischer-Tropsch synthesis are generally distributed from methane to high boiling compounds according to the so called Schulz-Flory distribution, well known to those skilled in the art. The Schulz-Flory distribution is expressed mathematically by the Schulz-Flory equation:

$$W_i = (1 - \alpha)^2 i\alpha^{i-1}$$

where i represents carbon number, is the Schulz-flory distribution factor which represents the ratio of the rate of chain propagation to the rate of chain propagation plus the rate of chain termination, and $W_i$ represents the weight fraction of product of carbon number i. This equation shows that an increased results in a higher average carbon number of the $\alpha$ products. Higher $\alpha$ values are desirable when heavier products, such as diesel fuel are relatively more valuable than lighter products, such as naphtha.

The products produced by the catalyst and the process of this invention generally follow the Schulz-Flory distribution, except that this yield of methane is usually higher than expected from this distribution. This indicates that methane is apparently produced by an additional mechanism.

Catalysts promoted with alkali in the manner prescribed in this invention produce a product having a higher average carbon number than the products from non-alkali promoted catalysts. That is, the Schulz-Flory $\alpha$ for the product from an alkali promoted catalyst is higher than for a non-alkali promoted catalyst.

It is well known, and also shown in one of the following examples, that rhenium alone is a low activity catalyst for Fischer-Tropsch synthesis producing a product which is predominantly methane. On the other hand, cobalt is a well known catalyst for producing higher carbon number hydrocarbons. In U.S. Patent no.4,568,663, it has been shown that adding small amounts of rhenium to cobalt supported on titania improves the catalytic activity. In the present invention, it has been found that the hydrocarbon yield obtained by adding rhenium is surprisingly much larger for an alumina supported cobalt catalyst than that obtained from cobalt and rhenium on several other inorganic supports. The improved activity and increased selectivity to heavier hydrocarbons is followed by no deleterious effect on the selectivity to methane.

The catalyst and the process of this invention are further described in the following examples.

The invention will be further illustrated with reference to the accompanying drawings, in which:-

Figure 1 is a graph showing the effect of rhenium content on CO conversion using catalysts containing 12% cobalt;

Figure 2 is a graph showing the effect on CO conversion of adding rhenium to catalysts containing various amounts of cobalt on an alumina support;

Figure 3 is a typical graph of CO conversion as a function of time on stream for the process of this invention when operated using a slurry reaction;

Figure 4 is a gas chromatagram of a typical middle distillate and heavier liquid product from the process of this invention;

Figure 5 is a graph showing the effect of potassium to cobalt ratio on the Schulz-Flory $\alpha$ of the product produced using the catalyst of this invention; and

Figure 6 is a graph showing the effect of potassium to cobalt ratio on CO conversion when using the catalysts of this invention.

The following non-limiting examples describe the preparation of various catalysts and the results obtained from testing these catalysts for conversion of synthesis gas into hydrocarbons.

Before being tested, each catalyst was given a pretreatment consisting of reduction by passing hydrogen over the catalyst at a rate of $3000 cm^3/g/h$ while heating the catalyst at a rate of $1C°/min$ to $350°C$ and maintaining this temperature for 10 hours. In all the process tests except for Example 42, synthesis gas consisting of 33 vol% carbon monoxide and 67 vol% hydrogen was atmospheric pressure at temperatures of 185, 195 and $205°C$ according to the following schedule:

9 hr. 50 min at $195°C$
4 hr. 20 min at $205°C$
4 hr. 30 min at $185°C$
9 hr. 50 min at $195°C$

The flow rate of synthesis gas was 1680 $cm^3/g$ of catalyst/h. Products from the reactor wee sent to a gas chromatograph for analysis. Catalysts were compared based on the results over the period from 10 to 30 hours on stream.

## EXAMPLE 1

### CATALYST CONTAINING COBALT BUT NO RHENIUM

This example describes the preparation of a control cobalt catalyst which used for comparative purposes. This catalyst was prepared as follows:

A solution was prepared by dissolving 17.03 of cobalt nitrate, $Co(NO_3)_2.6H_2O$, and 0.76g of mixed rare earth nitrate, $RE(NO_3)_3$, where RE sands for rare earth with a composition of 66% $La_2O_3$, 24% $Nd_2O_3$, 8.2% $Pr_6O_{11}$, 0.7% $CeO_2$, and 1.1% other oxides (molycorp 5247), in 30 ml of distilled water. The total solution was added with stirring to 25g of Ketjen CK300 gamma-alumina which had been calcined for 10 hours at $500°C$. The prepared catalyst was then dried for 5 hours in an oven at a temperature of $115°C$. The dried catalyst was then calcined in air by raising its temperature at a heating rate of $1C°/minute$ to $300°C$ and holding at this temperature for 2 hours. The finished catalyst contained 13 wt% cobalt and 1wt% rare earth oxide with the remainder being alumina. The catalyst is referred to as preparation "a" in Table 1. The above procedure was repeated to produce preparation "b" catalyst in Table 1.

The results of the tests with this catalyst are shown in Table I. In this and the following tables, selectivity is defined as the percent of the carbon monoxide converted that goes to the indicated product.

TABLE I

| Temp. °C | Preparation | CO conversion % | $C_2$ + selectivity % | $CH_4$ selectivity % | $CO_2$ selectivity % |
|---|---|---|---|---|---|
| 185 | a | 7 | 91.1 | 7.2 | 1.7 |
| | b | 11 | 91.8 | 7.1 | 1.1 |
| 195 | a | 12 | 90.0 | 8.9 | 1.1 |
| | b | 18 | 90.2 | 9.0 | 0.8 |
| 205 | a | 21 | 87.7 | 11.3 | 1.0 |
| | b | 29 | 86.7 | 12.4 | 0.9 |

This example shows that a cobalt catalyst exhibits good selectivity to ethane and longer chain length hydrocarbons and low selectivity to methane and carbon dioxide.

EXAMPLE 2

CATALYST CONTAINING RHENIUM BUT NO COBALT

This example describes a rhenium catalyst prepared for comparative purposes. The procedure employed was the same as for Example 1 except that the solution contained 0.33g of perrhenic acid, $HReO_4$ as 82.5% aqueous solution, and 0.54g of rare earth nitrate to make 24ml of solution which then was added to 20g of calcined alumina. The finished catalyst contained 1 wt% rhenium and 1wt% rare earth oxide with the remainder being alumina.

The results of the tests with the catalyst of Example 2 are shown in Table II.

TABLE II

| Temp °C | CO Conversion % | $C_2$ + Selectivity % | $CH_4$ Selectivity % | $CO_2$ Selectivity % |
|---|---|---|---|---|
| 185 | 0.3 | 20 | 30 | 50 |
| 195 | 0.3 | 19 | 30 | 50 |
| 205 | 0.3 | 19 | 30 | 50 |

EXAMPLE 3

CATALYST CONTAINING RHENIUM BUT NO COBALT

Repetition of the procedure from Example 2, except that 0.83 g of perrhenic acid was used, gave a catalyst containing 4 wt% rhenium. The results of the tests with the catalyst of Example 3 are shown in Table III.

TABLE III

| Temp °C | CO Conversion % | $C_2$ + Selectivity % | $CH_4$ Selectivity % | $CO_2$ Selectivity % |
|---|---|---|---|---|
| 185 | 0.3 | 20 | 30 | 50 |
| 195 | 0.3 | 19 | 31 | 50 |
| 205 | 0.3 | 19 | 31 | 50 |

The results from Examples 2 and 3 show that catalysts containing rhenium but no cobalt have very low activity for producing the desired liquid hydrocarbons from synthesis gas. Furthermore, about half the product is carbon dioxide, and most of the hydrocarbon product is methane.

EXAMPLES 4 TO 11

CATALYSTS CONTAINING BOTH COBALT AND RHENIUM

The preparation procedure of Example 1 was employed except that varying amounts of perrhenic acid were added to the solution. This produced a series of catalysts containing 12 wt% cobalt and 0.1, 0.2, 0.3, 0.5, 1.0, 2.0, 4.0, and 8.0 wt% rhenium in addition to 1.0 wt% rare earth oxide.

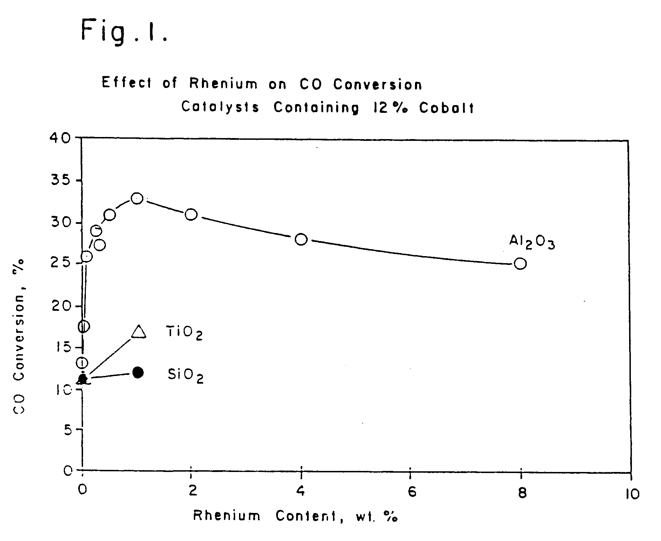

The results of the tests with the catalysts of Examples 4 to 11 at 195°C are shown in Table IV and further illustrated in Figure 1. Figure 1 shows the effect on carbon monoxide conversion of adding rhenium to catalysts containing 12% cobalt.

TABLE IV

| Example No. | Co wt% | Re wt% | CO Conversion % | $C_2$ + Selectivity % | $CH_4$ Selectivity % | $CO_2$ Selectivity % |
|---|---|---|---|---|---|---|
| 4 | 12 | 0.1 | 26 | 89.8 | 9.6 | 0.6 |
| 5 | 12 | 0.2 | 29 | 88.9 | 10.4 | 0.7 |
| 6 | 12 | 0.3 | 27 | 88.2 | 11.0 | 0.8 |
| 7 | 12 | 0.5 | 31 | 88.3 | 10.9 | 0.8 |
| 8 | 12 | 1.0 | 33 | 87.7 | 11.4 | 0.9 |
| 9 | 12 | 2.0 | 31 | 85.7 | 13.3 | 1.0 |
| 10 | 12 | 4.0 | 28 | 84.7 | 14.2 | 1.1 |
| 11 | 12 | 8.0 | 25 | 84.5 | 14.2 | 1.3 |

As can be seen from a comparison of the results in Table 1 with Table IV and Figure 1, the addition of small amounts of rhenium to a cobalt supported on alumina catalyst significantly increases the conversion of the carbon monoxide in the feed. Levels of rhenium even as low as 0.1 wt% results in approximately doubling the CO conversion. The exact level of Re for optimum activity is very important, as the rate of carbon monoxide conversion increases rapidly at low rhenium addition levels, reaches a maximum and then decreases gradually at levels greater than 1 wt% rhenium. However, even at the highest rhenium level investigated (8%), a clear improvement in conversion is evident when compared to the catalyst not containing rhenium.

It is important that the increase in activity should occur without a corresponding increase in either the methane or the carbon dioxide selectivities. Table IV shows that the increase in carbon monoxide conversion is not accompanied by any substantial change in either the selectivities to methane or carbon dioxide. Thus, after rhenium addition, the principal reaction products are still desirable hydrocarbons.

EXAMPLES 12 TO 25

CATALYSTS CONTAINING BOTH COBALT AND RHENIUM

The preparation procedure of Example 1 was employed except that varying amounts of cobalt nitrate and perrhemic acid were added to the solution. This produced a series of catalysts containing from 3.0 to 40 wt% cobalt and from 0 to 4.0 wt% rhenium in addition to 1.0 wt% rare earth oxide.

The results of the test with the catalysts of Examples 12 to 25 at 195°C are shown in Table V.

TABLE V

| Example No. | Co wt% | Re wt% | CO Conversion % | $C_2$ + Selectivity % | $CH_4$ Selectivity % | $CO_2$ Selectivity % |
|---|---|---|---|---|---|---|
| 12 | 3 | 0.0 | 5 | 90.7 | 8.2 | 1.2 |
| 13 | 3 | 0.25 | 4 | 87.2 | 10.4 | 2.4 |
| 14 | 6 | 0.0 | 12 | 90.0 | 8.9 | 1.1 |
| 15 | 6 | 0.5 | 16 | 88.2 | 10.8 | 1.0 |
| 16 | 9 | 0.0 | 15 | 90.0 | 9.1 | 0.9 |
| 17 | 9 | 0.75 | 25 | 88.1 | 11.1 | 0.8 |
| 18 | 20 | 0.0 | 20 | 89.3 | 9.8 | 0.9 |
| 19 | 20 | 0.5 | 40 | 87.9 | 11.1 | 1.0 |
| 20 | 20 | 1.0 | 46 | 86.1 | 12.9 | 1.0 |
| 21 | 20 | 5.0 | 42 | 83.9 | 14.8 | 1.3 |
| 22 | 40 | 0.0 | 20 | 89.3 | 9.7 | 1.0 |
| 23 | 40 | 1.0 | 56 | 85.0 | 13.2 | 1.8 |
| 24 | 40 | 2.0 | 58 | 84.3 | 13.7 | 2.0 |
| 25 | 40 | 5.0 | 60 | 81.9 | 15.7 | 2.4 |

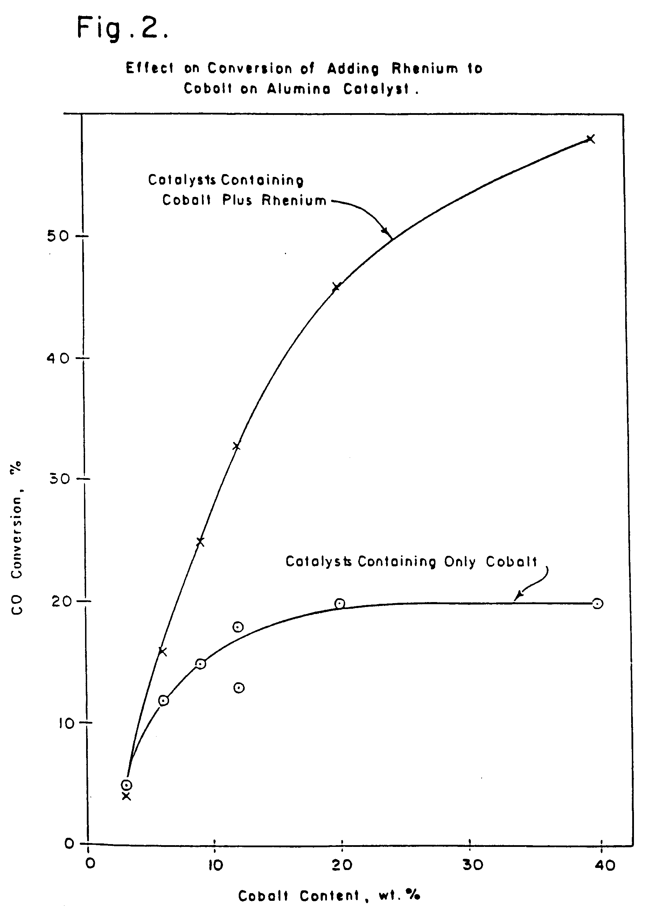

The results in Table V show that for cobalt catalysts without rhenium, there is a significant increase in activity in going from 3% cobalt to 6% cobalt. However, only modest increases in activity occur from this point up to cobalt loadings of as high as 40%. At a cobalt loading of 3%, the addition of rhenium does not improve the catalystic activity, but the improvement upon rhenium addition is significant for higher cobalt loadings. In fact, the improvement in activity due to the addition of rhenium increases as the cobalt content increases as shown in Figure 2.

EXAMPLES 26 AND 27

COBALT/RHENIUM CATALYSTS WITH PROMOTERS

To illustrate the use of promoters other than rare earth oxides, the following catalysts were prepared. The preparation procedure used to prepare the catalyst of Example 8 was used except that zirconium nitrate, $Zr(NO_3)_4$, or vanadyl oxalate, $VO(C_2O_4H)_3$, was substituted for the rare earth nitrate. The results of tests at 195°C with the catalysts of Examples 26 and 27 are shown in Table VI. In addition to the promoter, these catalysts contained 12% cobalt and 1% rhenium and were supported on alumina.

TABLE VI

| Example No. | Promoter | CO Conversion % | $C_2$ + Selectivity % | $CH_4$ Selectivity % | $CO_2$ Selectivity % |
|---|---|---|---|---|---|
| 26 | $ZrO_2$ (0.75wt%) | 31 | 87.9 | 11.3 | 0.8 |
| 27 | $V_2O_5$ (0.56wt%) | 26 | 89.4 | 9.8 | 0.8 |

EXAMPLES 28 TO 41

COBALT/RHENIUM CATALYSTS ON OTHER SUPPORTS

For comparison with alumina, several catalysts were prepared on other supports. The preparation procedure used to prepare the catalyst of Example 8 was repeated, but without the addition of a rare earth oxide. The titanium-supported catalysts were prepared on titania calcined at both 500°C and 600°C. After calcination at 600°C, the titania is mainly in the crystalline rutile form; while after calcination at 500°C the anatase:rutile ratio is about 1:1. The catalysts prepared on the titania support calcined at these two temperatures showed exactly the same catalytic activity.

The supports used were: Davison Grade 59 silica; Degussa P25 titania; Alpha Chemicals No.88272 chromia, magnesia prepared by calcining Fischer basic magnesium carbonate; American Cyanamid AAA Silica-Alumina; and Alpha Chemicals 11852 zirconia (containing 2% alumina). Information of the composition of the catalysts prepared on the different supports is given in Table VII.

EP 0 313 375 B1

TABLE VII

| Example No. | Support | Weight of Support | Weight of materials in impregnating | | Composition of finished catalyst wt% | |
|---|---|---|---|---|---|---|
| | | | $Co(NO_3)_2$ | $HReO_4$* | Co | Re |
| 28 | Silica | 20 | 13.47 | - | 12 | - |
| 29 | Silica | 20 | 12.62 | 0.38 | 12 | 1.0 |
| 30 | Titania** | 25 | 16.84 | - | 12 | - |
| 31 | Titania** | 24.64 | 16.78 | 0.46 | 12 | 1.0 |
| 32 | Titania*** | 25 | 16.84 | - | 12 | - |
| 33 | Titania*** | 24.64 | 16.78 | 0.46 | 12 | 1.0 |
| 34 | Chromia | 20 | 13.47 | - | 12 | - |
| 35 | Chromia | 21.3 | 14.51 | 0.40 | 12 | 1.0 |
| 36 | Magnesia | 21.59 | 14.54 | - | 12 | - |
| 37 | Magnesia | 14.54 | 10.67 | 0.29 | 12 | 1.0 |
| 38 | Silica-Magnesia | 20 | 13.47 | - | 12 | - |
| 39 | Silica-Magnesia | 20 | 13.62 | 0.38 | 12 | 1.0 |
| 40 | Zirconia | 20 | 13.47 | - | 12 | - |
| 41 | Zirconia | 20 | 13.62 | 0.38 | 12 | 1.0 |

* Weight of 82.5% perrhenic acid solution
** Calcined at 500°C
*** Calcined at 600°C.

A series of tests was conducted to evaluate the activities of the catalysts of the above examples in converting synthesis gas into hydrocarbons. The results of the tests with the catalysts of Example 28 to 41 at 195°C are shown in Table VIII. The results from catalysts prepared an alumina are included for comparison.

TABLE VIII

| Example No. | Co % | Re | Support | CO Conversion wt% | $C_2$ + Selectivity % | $CH_4$ Selectivity % | $CO_2$ Selectivity % |
|---|---|---|---|---|---|---|---|
| 1 | 12 | - | $Al_2O_3$ | 12 | 90.0 | 8.9 | 1.1 |
| 8 | 12 | 1 | $Al_2O_3$ | 33 | 87.7 | 11.4 | 0.9 |
| 28 | 12 | - | $SiO_2$ | 11 | 90.1 | 8.7 | 1.2 |
| 29 | 12 | 1 | $SiO_2$ | 12 | 88.1 | 10.7 | 1.2 |
| 30 | 12 | - | $TiO_2$* | 11 | 87.6 | 11.6 | 0.6 |
| 31 | 12 | 1 | $TiO_2$* | 17 | 86.5 | 12.8 | 0.7 |
| 32 | 12 | - | $TiO_2$** | 11 | 87.5 | 11.7 | 0.7 |
| 33 | 12 | 1 | $TiO_2$** | 17 | 85.8 | 13.5 | 0.7 |
| 34 | 12 | - | $Cr_2O_3$ | 1 | 83.5 | 15.5 | 1.0 |
| 35 | 12 | 1 | $Cr_2O_3$ | 2 | 80.8 | 12.3 | 6.9 |
| 36 | 12 | - | MgO | 0.3 | 20.0 | 30.0 | 50.0 |
| 37 | 12 | 1 | MgO | 0.3 | 19.1 | 30.9 | 50.0 |
| 38 | 12 | - | $SiO_2/Al_2O_3$ | 5 | 76.3 | 22.2 | 1.5 |
| 39 | 12 | 1 | $SiO_2/Al_2O_3$ | 6 | 78.6 | 19.8 | 1.6 |
| 40 | 12 | - | $ZrO_2$ | 4 | 80.9 | 16.3 | 2.8 |
| 41 | 12 | - | $ZrO_2$ | 7 | 78.8 | 18.7 | 2.5 |

* Support calcined at 500°C
** Support calcined at 600°C.

The catalysts in Table VII were prepared to test the teaching that various inorganic supports are acceptable for preparing cobalt plus rhenium F-T catalysts. An examination of the data in Table VIII leads to the surprising conclusion that the type of support is extremely important and that vast differences in activity

12

exist between catalysts prepared on one support and catalysts of the same catalytic metals content on another support. More surprisingly, only cobalt plus rhenium on alumina showed a commercially attractive activity level and selectivity.

Catalysts on magnesia and chromia exhibited extremely low activities, both with and without rhenium. Catalysts on zirconia and silica-alumina showed somewhat higher activities, but selectivity to $C_2+$ hydrocarbons was poor. These catalysts showed only modest improvements in activity upon the addition of rhenium.

Catalysts without rhenium supported on silica and titania showed activity levels close to comparable cobalt on alumina catalyst. However, upon addition of rhenium, the alumina catalyst showed a surprising increase in activity from about 15% carbon monoxide conversion to 33% carbon monoxide conversion; whereas, the silica supported catalyst showed only a very small increase in activity from 11% carbon monoxide conversion to 12% carbon monoxide conversion, while the titania supported catalyst showed a larger, but still modest, gain in activity from 11% carbon monoxide conversion to 17% carbon monoxide conversion.

From these examples and those presented previously, it can be concluded that the catalytic activity of a cobalt catalyst supported on alumina is greatly improved by adding minor amounts of rhenium, as long as the cobalt level is greater than about 3 wt%. Although improved activity from rhenium addition is also observed for some other supports, the activity level achieved by adding rhenium to a catalyst supported on alumina is much higher than for other supports. This result is surprising and would not have been predicated based on teachings in the prior art.

EXAMPLE 42

A catalyst was prepared according to the procedure of Example 24, except that Harshaw 4100 P alumina was used as the support. Before being tested in a slurry reactor, the catalyst was pretreated as follows. One hundred grams of this catalyst was loaded into a vertical pipe pretreating reactor constructed of 5ft (1.52m) of 1 in (2.5mm) OD schedule 40 stainless steel pipe. Hydrogen was introduced to the bottom of the pipe reactor at a rate of 1900 Sec/min which was sufficient to fluidise the catalyst in the pretreating reactor. After a fluidised bed was established, the temperature was increased at the rate of 1C°/min to a maximum temperature of 350°C. The catalyst was held at this temperature for 16 hr and then cooled to 50°C. At this point, the hydrogen was replaced with He, and the catalyst was cooled to ambient temperature. 14.1g of this reduced catalyst was mixed with 206 grams of Synfluid (Synfluid 8 cSt PAO, Chevron Chemical Company) and loaded into a 1 in (2.5mm) ID by 3ft (0.9m) slurry reactor. A mixture of CO, $H_2$, and $N_2$ in a ratio of 1:2:3 was fed to the reactor at a rate of 1080 Sl/hr. The temperature was increased to 225°C and the pressure was increased to 450 psig (31atm). These conditions were maintained for a total of 388 hr, except for two periods, one of 68 hrs and the other of 94hrs during which pure $N_2$ was fed to the reactor. After a transition period of 2 hours, there then followed a period of 90 hours during which the ratio of CO:$H_2$:$N_2$ in the feed was 1:3:4. There then followed a period of 53 hours during which operation was unstable due to poor temperatures control. During this period some high temperatures were experienced, followed by a short period of no activity from which the reactor quickly recovered. It is postulated that during the high temperature period, an abundance of light hydrocarbons were formed which diluted the vehicle liquid and caused collapse of the slurry bed. This illustrates the importance of maintaining the proper vehicle properties, mixing energy, etc. It also illustrates the inherent ruggedness of the process, since it was able to recover from this incident. This was followed by 160 hours during which the ratio of CO:$H_2$:$N_2$ in the feed was 1:1:2. After a 7 hour transition period, this was followed by a period of 115 hours in which the ratio of CO:$H_2$:$N_2$ in the feed was again 1:2:3. After a 9 hour transition period, there then followed a period of 157 hours with a CO:$H_2$:$N_2$ ratio in the feed gas of 2:3:5. Finally, after a 5 hour transition period, there was a period of 94 hours with a CO:$H_2$:$N_2$ feed ratio of 2:5:7. Altogether from startup to shutdown, the run lasted 1080 hours.

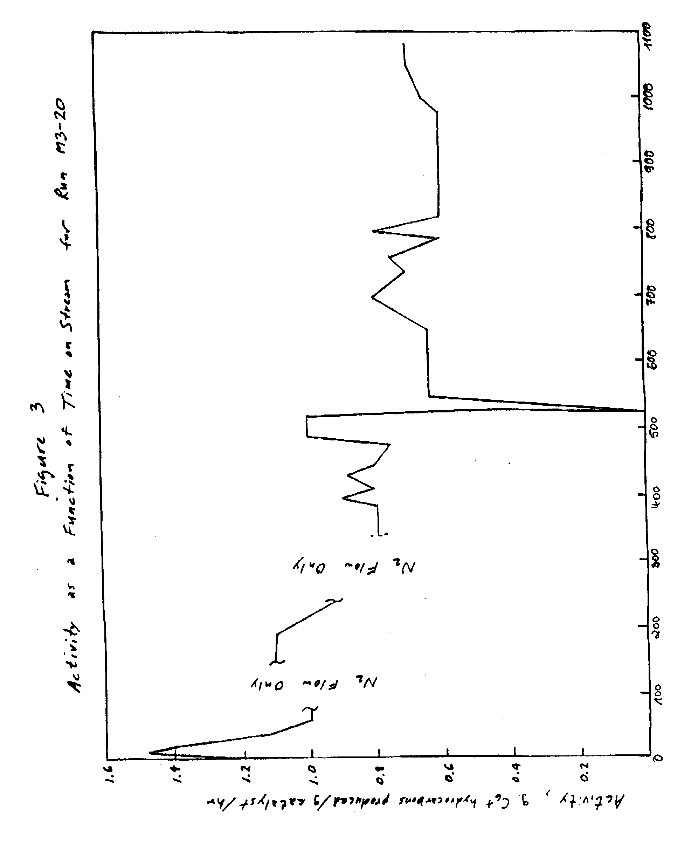

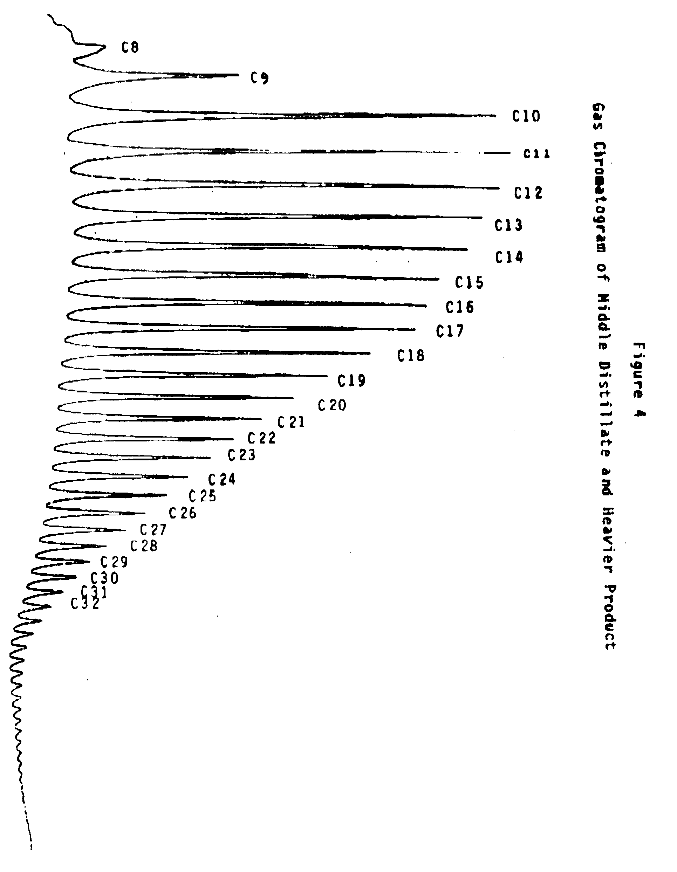

Figure 3 is a graph of CO conversion as a function of time on stream. This graph demonstrates the stability of the process and shows that the rate of deactivation is low. During operation, product was removed continuously from the unit, so that the slurry volume in the reactor remained constant. Figure 4 is a gas chromatogram of a sample of the middle distillate and heavier liquid product. The product is predominately normal paraffins with a typical Schulz-Flory distribution, showing that the catalyst is promoting the Fischer-Tropsch reaction.

This example demonstrates that the catalyst of this invention is active for the Fischer-Tropsch process. Moreover, it shows that the catalyst may be used in a slurry reactor, as well as in a fixed bed reactor, as illustrated by the previous examples.

EXAMPLES 3 TO 7

CATALYST CONTAINING COBALT, RHENIUM AND ALKALI

To demonstrate the advantages of including an alkali in the catalysts of this invention, the catalysts of Examples 3 to 7 were prepared and tested.

The catalysts of Examples 43 to 51 were prepared on Harshaw Al 4100P alumina which had been screened to 100-270 mesh. The catalysts of Examples 2 to 7 were prepared on Ketjen CK 300 gamma alumina, screened to 20 = 40 mesh. Both supports were calcined overnight at 500°C before use. The technique used for preparing the catalysts was incipinet wetness, as descrived in Example 1. The amounts of material used in the preparation of each catalyst are shown in Table IX. The prepared catalysts were then air dried for 5 to 24 hours in an oven at a temperature of 120°C. The dried catalysts were then calcined in air by raising the temperature at a heating rate of 1C°/minute to 300°C and held at this temperature for 2 to 16 hours. The compositions of the finished catalysts are shown in Table IX.

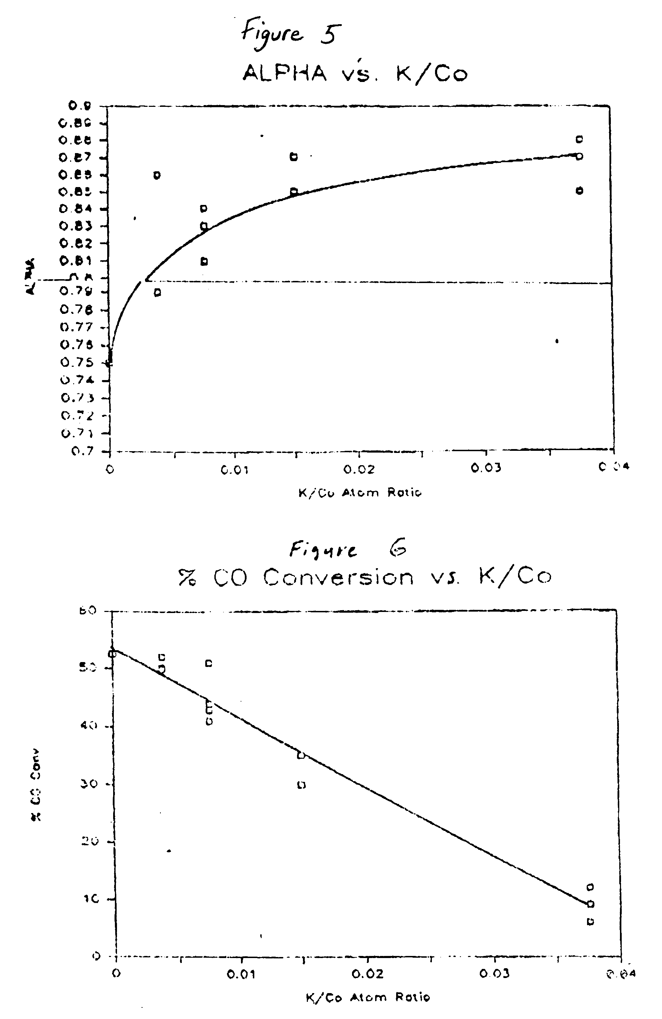

The results from testing these catalysts are shown in Table X. As is clear from these results, the addition of an alkali to the catalyst serves to increase the average molecular weight of the product, as evidenced by an increase in the Shulz-Flory $\alpha$. Higher levels of the alkali result in higher $\alpha$ as illustrated in Figure 5. However, activity decreases as the alkali content increases, as illustrated in Figure 6. Thus, for any particular situation there is an optimum alkali level that balances the desired average product molecular weight and catalyst activity. Also, the effectiveness of the alkali varies from one alkali to another with, for example, potassium being more effective than lithium.

TABLE IX

| Example No. | Wt of $Al_2O_3$ g | Type of Alkali | Weight of material in impregnation solution g | | | | Composition of finished catalyst wt% | | | |
|---|---|---|---|---|---|---|---|---|---|---|
| | | | (a) | (b) | (c) | (d) | Co | Re | REO | Alk |
| 43 | 300.0 | - | 1039.65 | 17.21 | 13.99 | - | 40 | 2.0 | 1.0 | 0.0 |
| 44 | 75.0 | K | 260.46 | 4.31 | 3.50 | 0.34 | 40 | 2.0 | 1.0 | 0.1 |
| 45 | 75.0 | K | 256.36 | 4.25 | - | 0.67 | 40 | 2.0 | - | 0.2 |
| 46 | 175.0 | K | 609.03 | 10.08 | 8.20 | 1.59 | 40 | 2.0 | 1.0 | 0.2 |
| 47 | 100.0 | K | 349.50 | 5.79 | 4.70 | 1.83 | 40 | 2.0 | 1.0 | 0.4 |
| 48 | 100.0 | K | 354.03 | 5.86 | 4.76 | 4.64 | 40 | 2.0 | 1.0 | 1.0 |
| 49 | 60.0 | Na | 205.49 | 3.40 | - | 0.93 | 40 | 2.0 | - | 0.2 |
| 50 | 60.0 | Cs | 209.55 | 3.47 | - | 2.12 | 40 | 2.0 | - | 1.3 |
| 51 | 65.0 | Rb | 225.38 | 4.23 | - | 1.17 | 40 | 2.0 | - | 0.8 |
| 52 | 20.0 | - | 13.78 | 0.38 | 0.62 | - | 12 | 1.0 | 1.0 | - |
| 53 | 20.0 | K | 13.80 | 0.38 | 0.62 | 0.06 | 12 | 1.0 | 1.0 | 0.1 |
| 54 | 20.0 | Li | 13.64 | 0.38 | - | 0.11 | 12 | 1.0 | - | 0.0 |
| 55 | 20.0 | Li | 13.80 | 0.38 | 0.62 | 0.12 | 12 | 1.0 | 1.0 | 0.0 |
| 56 | 20.0 | Cs | 13.67 | 0.38 | - | 0.10 | 12 | 1.0 | - | 0.3 |
| 57 | 20.0 | Cs | 13.83 | 0.38 | 0.62 | 0.10 | 12 | 1.0 | 1.0 | 0.2 |

(a) $Co(NO_3)_2.6H_2O$

(b) 82.5% $HReO_4$ solution, except for Example 50 which was 72.9 $HReO_4$

(c) Rare earth nitrates (see Example 1)

(d) $LiNO_3$, $NaNO_3$, $KNO_3$, $RbNO_3$, or $CsNO_3$

TABLE X

| Example No. | Alkali Type | Content wt% | CO Conversion % | CH$_4$ Selectivity % | Product alpha* |
|---|---|---|---|---|---|
| 43 | – | – | 52.5** | 14.2** | 0.75** |
| 44 | K | 0.1 | 52 | 8.6 | 0.86 |
|  |  |  | 50 | 11.1 | 0.79 |
| 45 | K | 0.2 | 43 | 9.3 | 0.83 |
| 46 | K | 0.2 | 51 | 9.5 | 0.84 |
|  |  |  | 44 | 11.6 | 0.84 |
|  |  |  | 41 | 9.4 | 0.81 |
| 47 | K | 0.4 | 30 | 7.3 | 0.87 |
|  |  |  | 35 | 7.8 | 0.85 |
| 48 | K | 1.0 | 12 | 7.0 | 0.88 |
|  |  |  | 9 | 7.9 | 0.85 |
|  |  |  | 6 | 6.2 | 0.87 |
| 49 | Na | 0.24 | 21 | 8.3 | 0.82 |
| 50 | Cs | 1.36 | 14 | 8.5 | 0.86 |
| 51 | Rb | 0.87 | 11 | 7.3 | 0.86 |
| 52 | – | – | 31.7*** | 10.9*** | 0.77*** |
| 53 | K | 0.1 | 19 | 7.6 | 0.78 |
|  |  |  | 22 | 9.8 | 0.84 |
| 54 | Li | 0.05 | 31 | 11.7 | 0.78 |
| 55 | Li | 0.05 | 27 | 12.0 | 0.78 |
| 56 | Cs | 0.3 | 21 | 11.1 | 0.83 |
| 57 | Cs | 0.3 | 21 | 10.3 | 0.84 |

*   Calculated by plotting $\ln(W_n/n)$ vs. n, where n is carbon number and $W_n$ is the weight fraction of the

```
        product having a carbon number n, and determining
        the slope of the line.
    **    Average of 21 tests
    ***    Average of 6 tests
```

## Claims

1. A catalyst for converting synthesis gas to hydrocarbons characterised in that it comprises catalytically active amounts of cobalt and relatively lesser amounts of rhenium composited on an alumina support.

2. A catalyst as claimed in Claim 1 characterised in that the cobalt is present in an amount ranging from 5 to 60 wt%, preferably 10 to 40wt% of the catalyst.

3. A catalyst as claimed in Claim 1 or Claim 2 characterised in that the rhenium is present in an amount ranging from 0.5 to 50wt%, preferably 1 to 30wt% of the cobalt content of the catalyst.

4. A catalyst as claimed in any preceding claim, characterised by the inclusion of an alkali metal promoter.

5. A catalyst as claimed in Claim 4, characterised in that the alkali metal promoter is present in a smaller amount than the cobalt content of the catlayst, preferably in amounts ranging from 0.5 to 5 atom % of the cobalt content of the catalyst.

6. A catalyst as claimed in Claims 1 to 5 for converting synthesis gas to hydrocarbons, characterised in that it comprises cobalt; rhenium and an alkali metal promoter composited on an alumina support wherein cobalt is present in catalytically active amounts up to 60wt% of the catalyst, rhenium is present in amounts from 0.5 to 50wt% of the cobalt content of the catalyst and the alkali is present in amounts from 0.5 to 5 atom percent of the cobalt content of the catalyst.

7. A catalyst as claimed in any of Claims 4 to 6 characterised in that the alkali is incorporated into the catalyst as a salt selected from the group consisting of nitrates, chlorides, carbonates and hydroxides.

8. A catalyst as claimed in any preceding claim characterised in that the support has surface area of at least $100m^2/g$ or $150m^2/g$ and a pore volume of at least $0.3cm^3/g$.

9. A catalyst as claimed in any preceding claim characterised by further comprising a promoter comprising an oxide of an element chosen from group IIIB, IVB or VB of the periodic table, including the lanthanides and the actinides, MgO or MnO, or mixtures thereof.

10. A catalyst as claimed in Claim 9 characterised in that the metal oxide promoter is present in an amount ranging from 0.1 to 5wt% of the catalyst.

11. A catalyst as claimed in any preceding claim characterised in that the support is gamma alumina.

12. A catalyst as claimed in any preceding Claim, characterised in that the sulphur content of the alumina support is kept to levels below about one hundred parts per million.

13. A process for the production of hydrocarbons from synthesis gas, characterised by passing the synthesis gas over a catalyst as claimed in any preceding claim.

14. A process as claimed in Claim 13 for the production of hydrocarbons comprising the step of contacting a synthesis gas feed including hydrogen and carbon monoxide over a catalyst, characterised in that the catalyst comprises cobalt and rhenium composited on an alumina support wherein rhenium is present in relatively lesser amounts than the cobalt content of the catalyst, at a temperature in the range of 150

16

to 300°C, a pressure in the range of 1.01 to 101 bars (atmospheric to 100 atmospheres) and a gaseous hourly space velocity, based on the total amount of synthesis gas feed, in the range of 100 to 20,000cm$^3$ of gas per gram of catalyst per hour.

15. A process as claimed in Claim 13 or Claim 14 characterised in that the temperature is in the range of 190 to 280°C.

16. A process as claimed in any of Claims 13 to 15 characterised in that the pressure is in the range of 1.01 to 40.53 bars (1 to 40 atmospheres).

17. A process as claimed in any of Claims 13 to 16, characterised in that the gaseous hourly space velocity is in the range of 100 to 10,000 cm$^3$ of gas per gram of catalyst per hour.

18. A process as claimed in any of Claims 13 to 17, characterised in that the synthesis gas feed is heated before contacting the catalyst.

19. A process as claimed in any of Claims 13 to 18, characterised in that the contacting step takes place in a fixed bed reactor, a fluidised bed reactor, an obullating bed reactor, or in a slurry reactor.

20. A process as claimed in any of Claims 13 to 19, characterised in that the molar ratio of hydrogen to carbon monoxide is between 1:1 to 3:1 and preferably, is between 1.5:1 to 2.5:1.

21. A process as claimed in any of Claims 13 to 20 characterised by the further step of cooling the gaseous product from the contacting step to a liquid by exposure to progressively lower temperatures.

## Patentansprüche

1. Katalysator für die Umwandlung von Synthesegas zu Kohlenwasserstoffen, dadurch gekennzeichnet, daß er katalytisch wirksame Mengen an Kobalt und relativ geringere Mengen an Rhenium enthält, die auf einem Aluminiumoxidträger verbunden sind.

2. Katalysator nach Anspruch 1, dadurch gekennzeichnet, daß das Kobalt in einer Menge von 5 bis 60 Gewichtsprozent des Katalysators, bevorzugt von 10 bis 40 Gewichtsprozent des Katalysators, vorliegt.

3. Katalysator nach Anspruch 1 oder Anspruch 2, dadurch gekennzeichnet, daß das Rhenium in einer Menge von 0,5 bis 50 Gewichtsprozent des Kobaltgehalts im Katalysator vorliegt.

4. Katalysator nach einem der vorausgehenden Ansprüche, gekennzeichnet durch den Einschluß eines Alkalimetallpromotors.

5. Katalysator nach Anspruch 4, dadurch gekennzeichnet, daß der Alkalimetallpromotor in geringeren Mengen als der Kobaltgehalt im Katalysator vorliegt, bevorzugt in einer Menge im Bereich von 0,5 bis 5 Atomprozent des Kobaltgehaltes des Katalysators.

6. Katalysator nach den Ansprüchen 1 bis 5 zur Umwandlung von Synthesegas zu Kohlenwasserstoffen, dadurch gekennzeichnet, daß er Kobalt, Rhenium und einen Alkalimetallpromtor enthält, die auf einem Aluminiumoxidträger verbunden sind, worin Kobalt in katalytisch aktiven Mengen bis 60 Gewichtsprozent des Katalysators, Rhenium in Mengen von 0,5 bis 50 Gewichtsprozent des Kobaltgehaltes des Katalysators und das Alkali in Mengen von 0,5 bis 5 Atomprozent des Kobaltgehalts des Katalysators vorliegen.

7. Katalysator nach einem der Ansprüche 4 bis 6, dadurch gekennzeichnet, daß das Alkali als Salz, das aus der Gruppe ausgewählt ist, die Nitrate, Chloride, Carbonate und Hydroxide umfaßt, eingelagert ist.

8. Katalysator nach einem der vorausgehenden Ansprüche, dadurch gekennzeichnet, daß der Träger eine Oberfläche von wenigstens 100m$^2$/g oder 150m$^2$/g und ein Porenvolumen von wenigstens 0,3cm$^3$/g besitzt.

**9.** Katalysator nach einem der vorausgehenden Ansprüche, dadurch gekennzeichnet, daß er weiterhin einen Promotor enthält, der ein Oxid eines Elements aus der IIIB-, IVB- oder VB-Gruppe des Periodensystems, einschließlich der Lanthaniden und der Actiniden, MgO oder MnO, oder Mischungen davon, enthält.

**10.** Katalysator nach Anspruch 9, dadurch gekennzeichnet, daß der Metalloxidpromotor in einer Menge im Bereich von 0,1 bis 5 Gewichtsprozent des Katalysators vorliegt.

**11.** Katalysator nach einem der vorausgehenden Ansprüche, dadurch gekennzeichnet, daß der Träger aus gamma-Aluminiumoxid besteht.

**12.** Katalysator nach einem der vorausgehenden Ansprüche, dadurch gekennzeichnet, daß der Schwefelgehalt des Aluminiumoxidträgers bei Werten von unter 100 ppm gehalten wird.

**13.** Verfahren zur Herstellung von Kohlenwasserstoffen aus Synthesegas, dadurch gekennzeichnet, daß das Synthesegas über einen Katalysator nach einem der vorausgehenden Ansprüche geleitet wird.

**14.** Verfahren nach Anspruch 13 zur Herstellung von Kohlenwasserstoffen, das den Schritt der Zuführung von Synthesegas, welches Wasserstoff und Kohlenmonoxid enthält, und dessen Führung über einen Katalysator zur Kontaktierung mit diesem enthält, dadurch gekennzeichnet, daß der Katalysator Kobalt und Rhenium enthält, die auf einem Aluminiumoxidträger verbunden sind, wobei Rhenium mit relativ geringeren Mengen vorliegt als der Kobaltgehalt im Katalysator, wobei die Temperatur im Bereich von 150°C bis 300°C, der Druck im Bereich von 1,01 bis 101 bar (von atmosphärisch bis 100 Atmosphären) und die stündlich zugeführte Menge an Gas, abhängig von der Gesamtmenge an zugeführtem Synthesegas, im Bereich von 100 bis 20.000 cm$^3$ Gas pro Gramm Katalysator und pro Stunde liegt.

**15.** Verfahren nach Anspruch 13 oder Anspruch 14, dadurch gekennzeichnet, daß die Temperatur im Bereich von 190° bis 280°C liegt.

**16.** Verfahren nach einem der Ansprüche 13 bis 15, dadurch gekennzeichnet, daß der Druck im Bereich von 1,01 bis 40,53 bar (1 bis 40 Atmosphären) liegt.

**17.** Verfahren nach einem der Ansprüche 13 bis 16, dadurch gekennzeichnet, daß die stündlich zugeführte Menge an Gas im Bereich von 100 bis 10.000 cm$^3$ Gas pro Gramm Katalysator und pro Stunde liegt.

**18.** Verfahren nach einem der Ansprüche 13 bis 17, dadurch gekennzeichnet, daß das zugeführte Synthesegas vor dem Kontakt mit dem Katalysator erwärmt wird.

**19.** Verfahren nach einem der Ansprüche 13 bis 18, dadurch gekennzeichnet, daß der Kontaktschritt in einem Festbettreaktor, in einem Fließbettreaktor, in einem Blasenbettreaktor oder in einem Suspensionsreaktor stattfindet.

**20.** Verfahren nach einem der Ansprüche 13 bis 19, dadurch gekennzeichnet, daß das Molverhältnis von Wasserstoff zu Kohlenmonoxid im Bereich von 1:1 bis 3:1 und bevorzugt im Bereich von 1.5:1 bis 2.5:1 liegt.

**21.** Verfahren nach einem der Ansprüche 13 bis 20, gekennzeichnet durch den weiteren Schritt einer Abkühlung des gasförmigen Produkts aus dem Kontaktschritt zu einem flüssigen Produkt dadurch, daß es zunehmend geringeren Temperaturen ausgesetzt wird.

**Revendications**

**1.** Catalyseur pour la conversion du gaz de synthèse en hydrocarbures, caractérisé en ce qu'il comprend des quantités catalytiquement actives de cobalt et des quantités relativement moindre de rhénium, combinées sur un support d'alumine.

**2.** Catalyseur selon la revendication 1, caractérisé en ce que le cobalt est présent en une concentration comprise entre 5 et 60 % en poids, de préférence entre 10 et 40 % en poids, relativement au

18

catalyseur.

**3.** Catalyseur selon la revendication 1 ou la revendication 2, caractérisé en ce que le rhénium est présent en une concentration comprise entre 0,5 et 50 % en poids, de préférence 1 à 30 % en poids de la teneur en cobalt du catalyseur.

**4.** Catalyseur selon l'une quelconque des revendications précédentes, caractérisé par l'incorporation d'un métal alcalin promoteur.

**5.** Catalyseur selon la revendication 4, caractérisé en ce que le métal alcalin promoteur est présent en une proportion inférieure à la teneur en cobalt du catalyseur, de préférence en des proportions comprises entre 0,5 et 5 % atomiques de la teneur en cobalt du catalyseur.

**6.** Catalyseur selon les revendications 1 à 5 pour la conversion du gaz de synthèse en hydrocarbures, caractérisé en ce qu'il comprend du cobalt, du rhénium et un métal alcalin promoteur combinés sur un support d'alumine, où le cobalt est présent en des proportions catalytiquement actives atteignant 60 % du poids du catalyseur, le rhénium est présent en des proportions de 0,5 à 50 % de la teneur en cobalt du catalyseur et le métal alcalin est présent en des proportions de 0,5 à 5 % atomiques de la teneur en cobalt du catalyseur.

**7.** Catalyseur selon l'une quelconque des revendications 4 à 6, caractérisé en ce que le métal alcalin est incorporé au catalyseur sous forme d'un sel choisi dans le groupe constitué des nitrates, chlorures, carbonates et hydroxydes.

**8.** Catalyseur selon l'une quelconque des revendications précédentes, caractérisé en ce que le support a une surface spécifique d'au moins 100 $m^2$/g ou 150 $m^2$/g et un volume des pores d'au moins 0,3 $cm^3$/g.

**9.** Catalyseur selon l'une quelconque des revendications précédentes, caractérisé en ce qu'il comprend de plus un promoteur comprenant un oxyde d'un élément choisi dans les groupes IIIB, IVB ou VB de la classification périodique, y compris les lanthanides et les actinides, MgO ou MnO, ou leurs mélanges.

**10.** Catalyseur selon la revendication 9, caractérisé en ce que l'oxyde métallique promoteur est présent en une proportion comprise entre 0,1 et 5 % du poids du catalyseur.

**11.** Catalyseur selon l'une quelconque des revendications précédentes, caractérisé en ce que le support est de la $\gamma$-alumine.

**12.** Catalyseur selon l'une quelconque des revendications précédentes, caractérisé en ce que la teneur en soufre du support d'alumine est maintenue à des teneurs inférieures à environ 100 parties par million.

**13.** Procédé pour la production d'hydrocarbures à partir du gaz de synthèse, caractérisé par le passage du gaz de synthèse sur un catalyseur selon l'une quelconque des revendications précédentes.

**14.** Procédé selon la revendication 13 pour la production d'hydrocarbures, comprenant l'étape de contact d'une alimentation de gaz de synthèse comprenant de l'hydrogène et du monoxyde de carbone sur un catalyseur, caractérisé en ce que le catalyseur comprend du cobalt et du rhénium combinés sur un support d'alumine, où le rhénium est présent en des proportions relativement inférieures à la teneur en cobalt du catalyseur, à une température dans la gamme de 150 à 300°C, à une pression dans la gamme de 1,01 à 101 bars (pression atmosphérique à 100 atmosphères) et à une vitesse spatiale horaire gazeuse, relative à la quantité totale de l'alimentation de gaz de synthèse, dans la gamme de 100 à 20 000 $cm^3$ de gaz par gramme de catalyseur par heure.

**15.** Procédé selon la revendication 13 ou la revendication 14, caractérisé en ce que la température est dans la gamme de 190 à 280°C.

**16.** Procédé selon l'une quelconque des revendications 13 à 15, caractérisé en ce que la pression est dans la gamme de 1,01 à 40,53 bars (1 à 40 atmosphères).

**17.** Procédé selon l'une quelconque des revendications 13 à 16, caractérisé en ce que la vitesse spatiale gazeuse horaire est dans la gamme de 100 à 10 000 cm³ de gaz par gramme de catalyseur par heure.

**18.** Procédé selon l'une quelconque des revendications 13 à 17, caractérisé en ce que l'alimentation de gaz de synthèse est chauffée avant le contact avec le catalyseur.

**19.** Procédé selon l'une quelconque des revendications 13 à 18, caractérisé en ce que l'étape de contact a lieu dans un réacteur à lit fixe, un réacteur à lit fluidisé, un réacteur à lit bouillonnant ou un réacteur à suspension.

**20.** Procédé selon l'une quelconque des revendications 13 à 19, caractérisé en ce que le rapport molaire de l'hydrogène au monoxyde de carbone est entre 1/1 et 3/1 et de préférence est entre 1,5/1 et 2,5/1.

**21.** Procédé selon l'une quelconque des revendications 13 à 20, caractérisé par l'étape complémentaire de refroidissement du produit gazeux de l'étape de contact en un liquide, par exposition à des températures progressivement plus basses.

## Fig.1.

Effect of Rhenium on CO Conversion
Catalysts Containing 12% Cobalt

# Fig.2.

### Effect on Conversion of Adding Rhenium to Cobalt on Alumina Catalyst.

Figure 3

Activity as a Function of Time on Stream for Run M3-20

Figure 4

Gas Chromatogram of Middle Distillate and Heavier Product

Figure 5
ALPHA vs. K/Co

K/Co Atom Ratio

Figure 6
% CO Conversion vs. K/Co

K/Co Atom Ratio